# EUROPEAN PATENT APPLICATION

(11) **EP 2 022 415 A1**
(43) Date of publication of application: **11.02.2009**
(21) Application number: 07015482.8
(22) Date of filing: 07.08.2007
(51) Int. Cl.: A61B 17/28, A61B 17/30, A61B 19/00

(54) **Pressure limiting forceps**

(71) Applicant: Lasner, Jeffrey I., Purchase NY 10577 (US); Lasner, Michael E., Mount Kisco NY 10549 (US)
(72) Inventor: Lasner, Jeffrey I., Purchase NY 10577 (US); Lasner, Michael E., Mount Kisco NY 10549 (US)
(74) Representative: Pintz, György

(57) **Abstract**

Forceps of the tips that are commonly used in surgical procedures having pressure limiting means that is effective after the tissue or material is firmly grasped by the platforms or tips, thus causing pressure being applied and competent damage to the tissue or material being handled by the forceps.

## Description

### Field of the Invention

This invention relates generally to forceps used in a surgical procedure relating to tissue and grafts having a construction which limits or reduces the pressure on the surgeon's hand while doing said procedure but maintaining a secure grasp with minimal pressure.

### Background of the Invention

In the surgical field, it is known that a problem exists in the handling of tissues and grafts with conventional forceps. When using currently available forceps the closing force is transmitted directly and proportionally to the tips of the forceps. As a consequence the surgeon needs to be aware of the pressure exerted on the tissue during manipulation. Too little pressure will result in slippage, and too much force applied to the forceps could result in tearing, puncturing, abrasion or scoring which is the primary reason for tissue breakdown and limited survivorship of surgical procedures, such as bypass and bowel resections.

When a surgeon uses a forceps it is generally held in the surgeon's non-dominant hand since the surgeon is normally concentrating and directing attention on what the dominant hand is doing so that it is clear that it is entirely possible to place an extra 20-30 grams of excess pressure on the very fragile tissue by means of the forceps. This excess pressure is transmitted directly to the tissue, thus causing significant trauma. This trauma causes the anatomic site to heal more slowly and break down over time, thus causing the ultimate failure of the surgery.

Several solutions have been suggested to solve the problem, which is proven by the large number of patents registered in this technical field. However, these patents do not imply the method according to the present invention. There is not even one patent that had close relation to our solution. However we still mention some patents that have shallow resemblance to our solution.

The US 2 532 406 A Patent teaches a singular band of material that is looped at the posterior end and not pivoted. It is meant to be very specific for holding and grasping and depends upon the convexity at the front end so that there is enough stabilization to reduce or eliminate flexion. In this case, flexion would cause an insecure grasp on anything meant to be held firmly.

The teaching of the US 3 768 126 A Patent depends upon a flattened and resilient shaft, not one that is convex and stiff. Clearly, a convex shaft would make the necessary compression (to open the platforms for operation) very difficult because part of the flexibility may come from the 'loop' at the distal end, but most of the flexibility comes from the flattened shafts themselves. Therefore, in this teaching, flattened and highly resilient shafts are necessary. This teaching does not include or depend upon front end pivoting.

The US 832 317 A Patent is very much the same as the teaching of US 2 532 406 A Patent. It is non-pivoted, made from a single piece of material and depends upon the convexities at the very front end for stiffness and stability.

The teaching of WO 98/00069 EK Patent is much like the teaching of US 3 768 126 A Patent. However, the US 3 768 126 A Patent appears to teach a handle that must be flat along the long axis (length) as well as within the short axis (width) in order to maintain proper flexibility. The cited WO 98/00069 EK Patent appears to teach a convexity along the long axis while being flat along the width. Also, the cited teaching is not pivoted.

The DE 201 16 266 U1 German Patent refers to a surgical instrument that is made from a forging, which is not flexibly resilient and does not depend upon a convexity along the inside of the shaft to create inflexibility. If this is a device made from a forging, and machined, which it does appear to be, it must be 'heat treated' to be hardened. Such an instrument or product would not depend upon the convexity for stiffness in order to properly transmit to the tips, an applied force to the handle. According to the attached drawings, the insides of the entire instrument are flattened and do not appear to have any convexities. The apparent round profile on the outside of the elongated shafts of this teaching is not structurally significant, since the inside is flattened. This round profile is significant only to the ergonomics of the use of the instrument in tight small surgical sites.

### Summary of the Invention

The present invention has been developed in order to overcome the propensity of surgeons to inadvertently apply excess pressure on forceps during surgical procedures resulting in tissue damage. In this regard the forceps are provided with means for limiting or reducing the pressure exerted on tissue by an arrangement of stress relief that is built into the instrument. Thus, the pressure exerted on the forceps is automatically limited or reduced by means of a flexible resilient arms attached to the main forceps construction that is made of relatively strong and inflexible stainless steel, or any other surgical quality material.

Furthermore, in the preferred embodiment of the invention, additional stress relief is obtained by means of the flexible interlocking sleeves at the distal end of the forceps. It should be noted, however, that the present forceps instrument will provide stress relief with the flexible arm or arms alone connected between the main body and the gripping platforms of the forceps.

The above and other features of the present invention will be apparent by reference to the following description of my invention together with the accompanying drawings.

### Brief Description of the Drawings

Fig. 1 is a perspective view of the pressure limiting forceps constructed in accordance with the teachings of my invention.
Fig. 1A is an enlarged partial perspective view of the forward end of the pressure limiting forceps shown in Fig. 1.
Fig. 2 is a side elevational view of the pressure limiting forceps in its natural inactive state.
Fig. 3 is another side elevational view of the pressure limiting forceps in an active state with the platforms of the forceps commencing to grasp tissue.
Fig. 4 is a further side elevational view of the forceps showing the handle of the forceps being further squeezed by the user's hands so that the tissue is firmly grasped at it's maximal pressure and
Fig. 5 is a top plan view of the pressure limiting forceps of the present invention.

### Description of the Preferred Embodiment

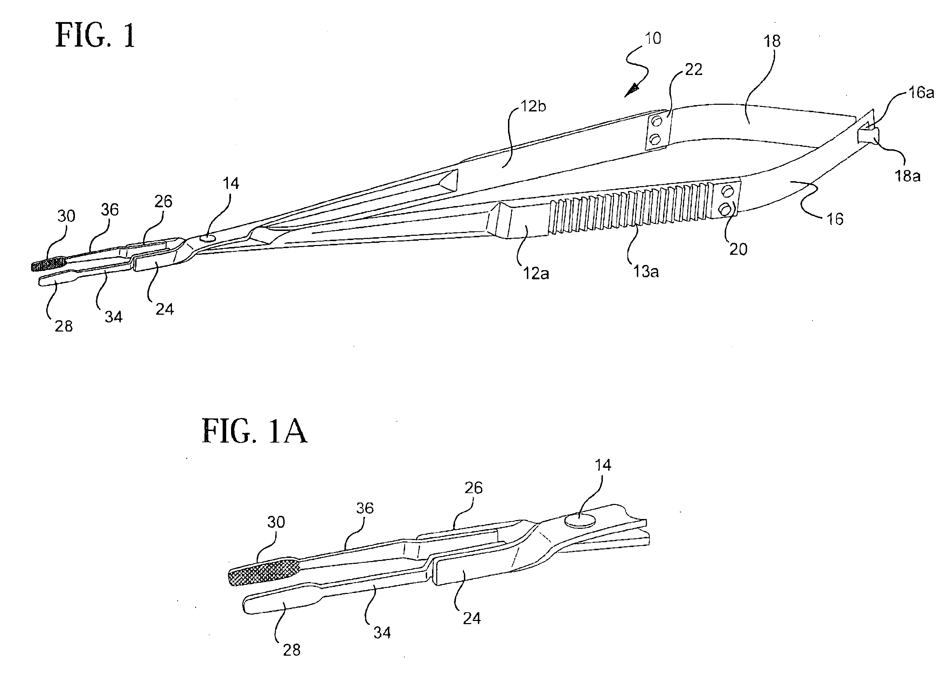

Forceps are presently used by health professionals, such as surgeons, in connection with procedures involving tissue manipulation. In order to ensure the proper amount of the pressure and the control a pressure limiting forceps is applied according to present invention, as shown in Figs 1-3 of the drawings. The main body 10 of the forceps includes a handle that is provided with two parts 12a and 12b respectively which are pivotally connected at the forward end of the forceps by means of pivot 14. The handles 12a and 12b are provided with slip resistant grasping surfaces 13a and 13b. The distal end of the forceps has flexible interlocking bands 16 and 18 each being attached at to the handles 12a and 12b by securing means 20 and 22. The other ends of the flexible bands 16 and 18 are male-female connected by means of projection 18a of band 18 free sliding in opening 16a of the band 16.

As seen in Fig. 1A, the forward end of the forceps in front of the pivot 14 have stops 24 and 26 made of strong and inflexible material. The gripping platforms or tips 28 and 30 are also fabricated of strong and inflexible material, such as stainless steel or any other surgical quality material. Furthermore, it is preferable to provide the gripping platforms 28 and 30 with a slip resistant surface, such as carbide interdigitating serrated tips.

Connected to the main body of the forceps are resilient arms 34 and 36 that are attached to the inside of stops 24 and 26. The arms 34 and 36 are constructed of highly flexible material with a high modulus of elasticity and low deformation probability.

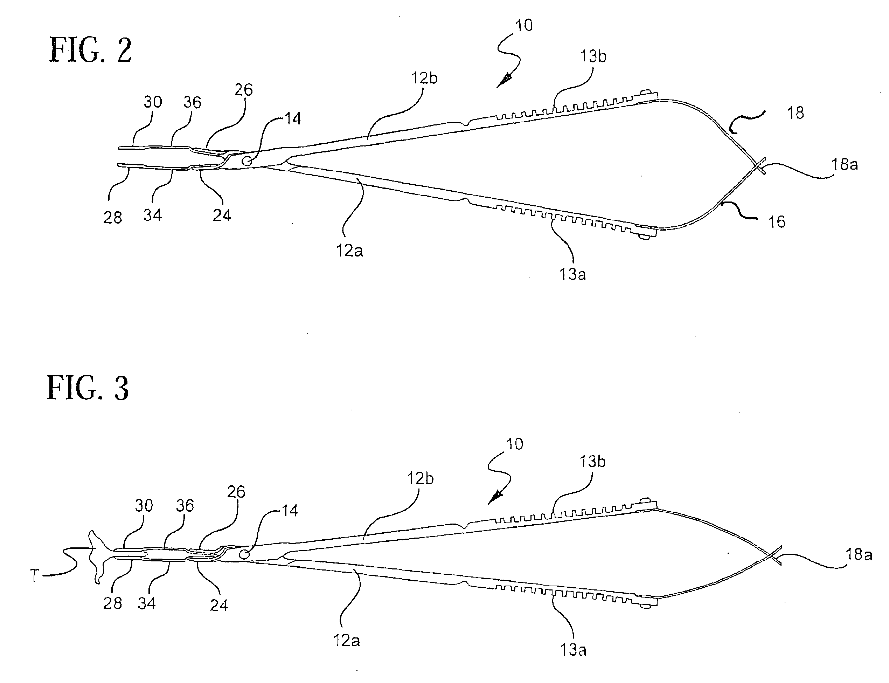

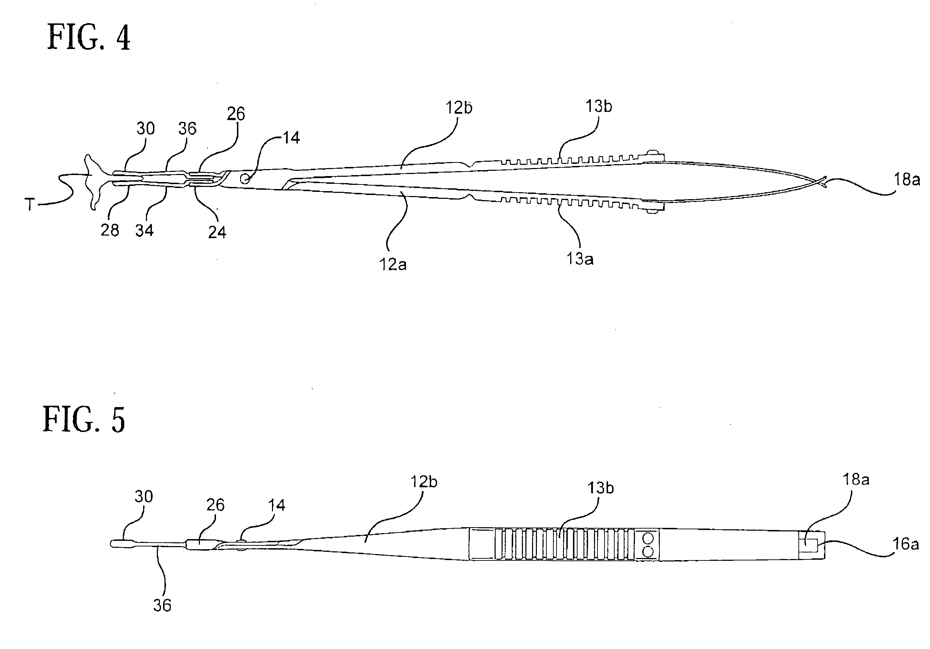

Referring now to Figs. 2-4, when a surgeon is using the pressure limiting forceps in his or her non-dominant hand during a surgical procedure while the dominant hand has a surgical instrument for carrying out the particular procedure, extreme care must be exercised on the amount of pressure exerted on the forceps grasping delicate tissue. With the present forceps in which the handle parts 12a and 12b are squeezed toward one another the tissue is grasped, as seen in Fig. 3. As seen in Fig. 4, further pressure is applied to the handles 12a and 12b when the handles are being squeezed closer together, and the gripping platforms 28 and 30 apply more pressure on the firmly grasped tissue continuously to a point just prior to complete forceps closure. At this point the stops 24 and 26 become effective through the connected ends of resilient arms 34 and 36.

When a stop arrangement is effected as seen as Fig. 4 the platforms of the forceps, which are firmly holding the tissue presented from further closure since arms 34 and 36 flex to relieve the excess pressure. In this case, the long axis of each of the arms 34 and 36 flex to the said point of complete forceps closure and continues to flex up to and including complete closure. When the platforms 28 and 30 are completely closed any additional pressure applied to the forceps results in stress relief by means of the flexible resilient arms 34 and 36, as well as by the flexible interlocking bands 16 and 18. Thus, the present arrangement results in a net pressure release on the tissue T. Excess pressure on the forceps is thus relieved on both the front and distal ends of the forceps.

A testing have been completed for the gripping force of the various devices. For each device, the forceps was clamped onto a Tekscan 6900 model sensor (Teksacan, Inc., South Boston, MA) which was encompassed with a latex sleeve. The pressure saturation for the sensor was 1100 pound per square inch (- 77,4 kg/cm²) and data were collected and analyzed with I-scan (Teksacan, Inc., South Boston, MA) software. Prior to testing, the new sensor was conditioned initially by applying known compressive forces with an Instron 8521S servohydraulic load frame (Instron Corp., Canton, MA). Then, a two-point linear calibration was performed with the I-scan software. Six trials were then recorded for each device to determine the maximum gripping force for each forceps. This testing were absolutely suitable to estimate the gripping forces of the different forceps devices.

Although the present described forceps is shown handling tissue in a surgical procedure, it should be apparent that the forceps can be used also wherever delicate material in any discipline must be handled to avoid tearing, puncturing or marring of the delicate or fragile material.

While the present invention has been disclosed and described with reference to a single embodiment, it will be apparent that changes and modifications may be made therein, and it is intended in the following claims to cover each variation and modification as falls within the true spirit and scope of the invention.

## Claims

1. Pressure limiting forceps primarily for handling tissues and other delicate materials especially for surgical application having an inflexible main body (10) and flexible interlocking bands (16, 18), and the main body (10) is provided by handles connected with a pivot (14) **characterized in that** arms (34, 36) are connected to the end opposite with the handles (12a, 12b) of the main body, at the one end of the arms (34, 36) there is a gripping platform (28, 30) while the other end together with the projection of the main body (10) constitutes a stop (24, 26).

2. A forceps according to claim 1, **characterized in that** at least one of the arms (34, 36) is fabricated of a highly flexible material.

3. A forceps according to claim 1 or 2, **characterized in that** the gripping platforms are of inflexible material further they are provided with slip resistant inner surface optionally with carbide interdigitating serrated tips.

4. A forceps according to any of claims 1-3, **characterized in that** the handles (12a, 12b) are provided with slip resistant grasping surfaces (13a, 13b).

5. A forceps according to any of claims 1-4, **characterized in that** the interlocking bands (16, 18) are attached to each other at the from handles (12a, 12b) distal ends of the interlocking bands (16, 18) with a slidable male-female connection by the help of an opening (16a) and a projection (18a).

6. A process for the application of the pressure limiting forceps according to claim 1 in the course of which primarily a surgical tissue (T) is grasped by the manual pressure of the handles (12a, 12b) **characterized in that** by the influence of the manual pressure the gripping platforms (28, 30) grasp the tissue (T) and lock on it while the stops (24, 26) totally meet, then a pressure relief is established partly by flexing of the arms (34, 36), partly by further flexing of the interlocking bands (16, 18), hereby in spite of the increase of the manual pressure the pressure on the tissue (T) does not increase.
